# FASCICULE DE BREVET EUROPEEN

(11) **EP 2 800 574 B1**
(45) Date de publication et mention de la délivrance du brevet: **09.08.2017**
(21) Numéro de dépôt: 12756729.5
(22) Date de dépôt: 07.09.2012
(51) Int. Cl.: A61K 35/64, A61K 36/87, A61P 29/00, A61K 31/192, A61K 31/352, A61K 45/06, A61K 36/18, A23L 21/20, A23L 33/105, A61K 35/644

(54) **COMPOSITIONS ANTI-INFLAMMATOIRES COMPRENANT DE LA MALVIDINE-3-O-BETA GLUCOSIDE ET UN EXTRAIT DE PROPOLIS**
ENTZÜNDUNGSHEMMENDE ZUSAMMENSETZUNGEN ENTHALTEND MALVIDIN-3-O-BETA GLUCOSIDE UND EINEN PROPOLIS EXTRAKT
ANTI-INFLAMMATORY COMPOSITIONS COMPRISING MALVIDIN-3-O-BETA GLUCOSIDE AND A PROPOLIS EXTRACT

(30) Priorité: 08.09.2011 FR 1157995
(43) Date de publication de la demande: 12.11.2014
(73) Titulaire: UNIVERSITE DE BORDEAUX, 33000 Bordeaux (FR); Nutrivercell, 91000 Evry (FR)
(72) Inventeur: RENARD, Loïc, F-92500 Rueil Malmaison (FR); MOSSALYAI, M. Djavad, F-33000 Bordeaux (FR); MERILLON, Jean-Michel, F-33650 Martillac (FR)
(74) Mandataire: Lavoix
(86) Numéro de dépôt international: PCT/EP2012/067584
(87) Numéro de publication internationale: WO 2013/034746

(56) Documents cités:
- CN-A- 1 994 357
- FR-A1- 2 916 141
- BORRELLI F ET AL: "Phytochemical compounds involved in the anti-inflammatory effect of propolis extract.", FITOTERAPIA, vol. 73, no. Supplement 1, novembre 2002 (2002-11), pages S53-S63, XP002669338, ISSN: 0367-326X cité dans la demande
- DATABASE WPI Week 200509 Thomson Scientific, London, GB; AN 2005-076593 XP002669339, & JP 2005 001998 A (API KK) 6 janvier 2005 (2005-01-06)

## Description

La présente invention concerne une composition anti-inflammatoire comprenant un extrait végétal comprenant une teneur spécifique en malvidine-3-O-β-glucoside et un extrait de propolis. L'invention concerne plus particulièrement l'utilisation de cette composition dans le traitement des maladies inflammatoires, notamment des arthrites.

La prise en charge actuelle des pathologies inflammatoires, et notamment de l'arthrite, est basée majoritairement sur l'utilisation d'agents anti-inflammatoires stéroïdiens (corticoïdes) ou d'agents anti-inflammatoires non stéroïdiens, tels que l'ibuprofène, le kétoprofène, l'acide niflumique ou le diclofénac.

Cependant, il existe des inconvénients majeurs liés à l'utilisation de ces agents anti-inflammatoires, notamment leurs effets néfastes sur l'estomac (ulcères gastriques ou duodénaux).

Par ailleurs, il existe des traitements de fond qui participent à l'extinction des symptômes de l'inflammation, tels que les sulfasalazines (Salazopyrine®).

Cependant, de nombreux inconvénients persistent avec ces traitements, notamment la toxicité à long terme des molécules telles que les corticoïdes mais également l'absence de prévention dans l'apparition de l'inflammation.

L'activité anti-inflammatoire de la propolis, notamment de l'acide caféique ou de son ester contenu dans la propolis est connue (Borelli et al, Fitoterapia (2002), 73 Suppl 1, 53-63).

Cependant, cette activité, lorsque la teneur en propolis est limitée, est insuffisante pour inhiber la réponse inflammatoire dans le cas de maladies inflammatoires chroniques telles que les arthrites.

L'activité anti-inflammatoire des anthocyanes, notamment de la malvidine et de son glucoside, est également connue (Wang et al, J.Agric.Food.Chem (2002), 50, 850-857).

Le document FR 2 916 141 décrit l'utilisation de la malvidine-3-O-β-glucoside dans une composition médicamenteuse pour prévenir ou traiter une pathologie impliquant des processus inflammatoires chroniques ou aigus, notamment la polyarthrite rhumatoïde.

Il n'existe cependant aujourd'hui pas de compositions atoxiques, notamment alimentaires ou topiques, présentant une activité anti-inflammatoire, notamment à l'encontre des arthrites, dont l'efficacité est proche voir équivalente aux agents anti-inflammatoires communément utilisés.

Ainsi un premier objectif de l'invention consiste à proposer une composition qui s'affranchisse des inconvénients de l'état de la technique et qui apporte une solution à tout ou partie des problèmes de l'état de la technique.

Un autre objectif est de proposer une composition anti-inflammatoire dont l'efficacité sur le traitement des pathologies impliquant des processus inflammatoires chroniques ou aigus, notamment de l'arthrite, est proche voire équivalente à celle des compositions actuellement disponibles.

Un autre objectif est de proposer une composition anti-inflammatoire atoxique, pouvant se présenter sous la forme d'une composition alimentaire ou à usage topique.

Un autre objectif de l'invention est de proposer une composition anti-inflammatoire pouvant être combinée à d'autres agents anti-inflammatoires, tels que la cortisone ou le methotréxate, diminuant ainsi les risques de toxicité liés à leur utilisation.

La présente invention a pour objet une composition anti-inflammatoire comprenant :
(a) un extrait végétal comprenant au moins 5% en poids, de préférence de 5 à 20% en poids, avantageusement de 5 à 15% en poids, de malvidine-3-O-β-glucoside ; et
(b) un extrait de propolis comprenant de l'acide caféique ou un de ses esters, de l'acide férulique, de la galangine et de la pinocembrine ;
en un ratio en poids a/b extrait végétal/extrait de propolis allant de 1/4 à 4/1.

La malvidine-3-O-β-glucoside est un dérivé glucoside de la malvidine ayant la formule suivante :

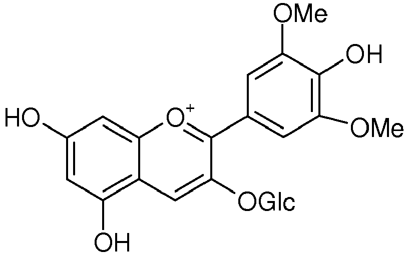

Selon l'invention, l'extrait végétal peut être choisi parmi les extraits de raisin, de myrtilles (*Vaccinium spp),* de mûres *(Rubus fruticosus)* ou encore de choux rouges *(Brassica oleracea* var. *capitata* f. *rubra*)*.*

Selon l'invention, l'extrait végétal est avantageusement choisi parmi les extraits de cuticule de raisin.

Selon l'invention, l'extrait de cuticule de raisin est obtenu ou susceptible d'être obtenu par un procédé comprenant la concentration des fractions polyphénoliques d'au moins un marc de raisin rouge.

Par marc de raisin, on entend l'ensemble que forment les cuticules, les pépins et la rafle obtenus du pressurage du raisin après les avoir séparés du moût.

Selon l'invention, le marc de raisin rouge est avantageusement issu du pressage de grappes de raisins issus des cépages Merlot, Cabernets tels que Sauvignon ou Franc, Syrah, Gamays, Grenache, Tannat ou Alicanthe Bouchet ou de leurs mélanges.

Selon l'invention, l'extrait de cuticule de raisin est avantageusement obtenu par un procédé comprenant la sélection d'au moins un marc de raisin rouge et la concentration des fractions polyphénoliques dudit marc.

Le procédé d'obtention de l'extrait de cuticule de raisin peut notamment comprendre une première étape de sélection de marcs de raisin rouge puis une diffusion dudit marc en présence d'une dispersion aqueuse de dioxyde de soufre.

On procède ensuite à une centrifugation, puis à une concentration des fractions polyphénoliques, notamment par évaporation, suivie d'une concentration sous vide.

Le produit ainsi obtenu peut subir une atomisation permettant ainsi d'obtenir un extrait de cuticule de raisin sous forme d'une poudre comprenant au moins 5% en poids de malvidine-3-O-β-glucoside, ainsi qu'une faible humidité résiduelle.

La proportion en malvidine-3-O-β-glucoside de l'extrait végétal est notamment mesurée par la méthode HPLC couplée à un spectrophotomètre UV-Visible DAD (détection à 535nm) en utilisant une colonne C18 phase inverse et un gradient de solvant composé d'acide trifluoroacétique et d'acétonitrile.

Comme exemple d'extrait de cuticules de raisin, on peut citer un extrait de cuticules de raisin comprenant 10% en poids de malvidine-3-O-β-glucoside commercialisé par la société GRAP'SUD sous la référence Anthos-vin.

La teneur en poids d'extrait végétal dans la composition selon l'invention va de 0,1 à 50%, de préférence de 1 à 40%.

La propolis désigne toute une série de substances résineuses, gommeuses et balsamiques, de consistance visqueuse, recueillies sur certaines parties, notamment les bourgeons et les écorces, de végétaux par les abeilles qui les transportent à la ruche et les modifient en partie avec l'ajout de leurs propres sécrétions salivaires et de cire. Ces végétaux sont principalement des arbres tels que pins, sapins, épicéas, peupliers, aulnes, saules, marronniers d'Inde, bouleaux, pruniers, frênes, chênes ou encore ormes.

Par extrait de propolis, on entend une forme de propolis pouvant être mise en oeuvre. Elle peut donc être non transformée ou traitée, ou bien transformée en présence notamment d'un excipient approprié, par exemple caroube, amidon ou dérivé d'amidon, e.g. maltodextrine. La propolis peut notamment se présenter sous la forme d'une poudre. Pour cela, la propolis peut être mélangée à une solution hydro alcoolique à laquelle est ajouté un excipient, tel que la maltodextrine ou la poudre de caroube, en tant que support. Le mélange ainsi obtenu est évaporé puis séché. Un extrait de propolis comprenant 18% de propolis et 82% de poudre de caroube est en outre commercialisé sous le nom propolis PPM 18 par la société LUSTREL. Un autre exemple d'extrait de propolis comprenant 60% de propolis et 40% de caroube est commercialisé par la société Plantex.

La teneur en poids d'extrait de propolis dans la composition selon l'invention va de 20 à 80%, de préférence de 30 à 70%.

Selon l'invention, l'extrait de propolis comprend une teneur en poids d'acide caféique ou d'un de ses esters de 0,001 à 0,2%, de préférence de 0,005 à 0,15%, d'acide férulique de 0,0001 à 0,005%, de préférence de 0,0005 à 0,003%, de galangine de 0,05 à 10%, de préférence de 0,1 à 6% et de pinocembrine de 0,1 à 5%, de préférence de 0,2 à 3%.

L'acide caféique ou acide (E) 3-(3,4-dihydroxyphényl)prop-2-ènoïque est un acide hydroxycarboxylique ayant la formule suivante :

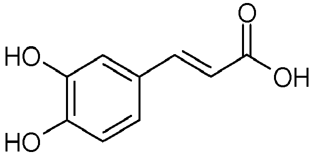

L'acide férulique ou acide 3-(4-hydroxy-3-méthoxyphényl)prop-2-ènoïque est un acide hydroxycarboxylique ayant la formule suivante :

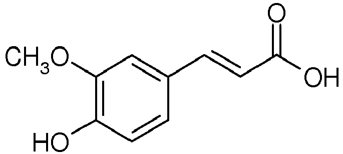

La galangine ou 3,5,7-trihydroxy-2-phenylchromèn-4-one est un dérivé flavonoïde ayant la formule suivante :

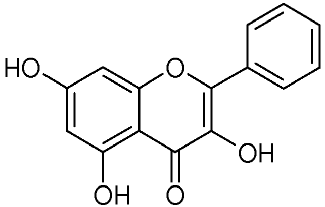

La pinocembrine ou (2*R*,3*R*)-3,5,7-Trihydroxy-2-phenyl-chroman-4-one est un dérivé flavonoïde ayant la formule suivante :

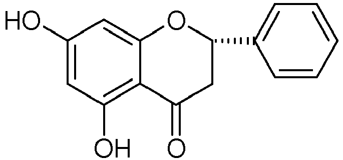

En outre, la composition selon l'invention peut également comprendre au moins un composé actif additionnel qui peut être choisi parmi les agents analgésiques, de préférence la capsaïcine, avantageusement issue de piments de Cayenne, les agents antibactériens, de préférence un extrait de cranberry, avantageusement un extrait de cranberry *Vaccinium macrocarpon,* les agents anti-inflammatoires ou leur mélange.

Dans un mode de réalisation, la composition selon l'invention comprend également de la capsaïcine ou un agent anti-inflammatoire ou leur mélange.

La teneur en poids de capsaïcine dans la composition selon l'invention va de 0,01 à 0,1%, de préférence de 0,025 à 0,075%.

Comme exemples d'extraits de cranberry, on peut citer ceux décrit dans le document WO 2011/020957, et notamment celui commercialisé par la société Tournay Biotechnologies sous la référence Exocyan CRAN 20S.

La teneur en poids dans la composition selon l'invention d'extrait de cranberry va de 5 à 40%.

En outre, la composition selon l'invention peut également comprendre un ingrédient alimentaire.

De manière avantageuse, l'ingrédient alimentaire est de la pipérine.

La pipérine peut être issue de différentes sources telles que des champignons ou du poivre noir, blanc ou gris.

De manière avantageuse, la pipérine est issue du poivre noir, blanc ou gris, de préférence du poivre noir. Comme exemple, on peut citer l'extrait sec de poivre noir commercialisé par la société Plantex comprenant de 10 à 15% de pipérine.

Sans être lié à aucune théorie en particulier, la pipérine est susceptible de favoriser l'absorption ainsi que la biodisponibilité des composés actifs présents dans la composition selon l'invention, notamment de la malvidine-3-O-β-glucoside, de l'acide caféique ou d'un de ses esters, de l'acide férulique, de la galangine et/ou de la pinocembrine, permettant ainsi d'améliorer l'efficacité anti-inflammatoire de la composition selon l'invention.

Selon l'invention, la teneur en poids de pipérine dans la composition selon l'invention va de 0,01 à 4%, de préférence de 0,1 à 1%.

L'ingrédient alimentaire peut également être choisi dans le groupe constitué par le zinc et ses dérivés.

Selon l'invention, les dérivés du zinc peuvent être choisis parmi les sels de zinc, tels que le citrate de zinc, l'oxyde de zinc, le sulfate de zinc, l'acétate de zinc ou le gluconate de zinc.

De manière avantageuse, le sel de zinc est le sulfate de zinc.

Selon l'invention, la teneur en poids de zinc ou d'un de ses dérivés dans la composition selon l'invention va de 0,01 à 2%, de préférence de 0,1 à 1,5%.

Un autre aspect de l'invention concerne la préparation d'une composition selon l'invention, comprenant
(i) la préparation d'un extrait végétal comprenant au moins 5% en poids, de préférence de 5 à 20% en poids, avantageusement de 5 à 15% en poids, de malvidine-3-O*-*β-glucoside,
(ii) le mélange de l'extrait issu de (i) et d'un extrait de propolis.

De manière avantageuse, l'invention concerne la préparation d'une composition selon l'invention, comprenant :
(i) la préparation d'un extrait de cuticule de raisin obtenu par la sélection d'au moins un marc de raisin rouge et la concentration des fractions polyphénoliques dudit marc, et
(ii) le mélange de l'extrait issu de (i) et d'un extrait de propolis.

De manière avantageuse, l'invention concerne la préparation d'une composition selon l'invention, comprenant :
(i) la préparation d'un extrait de cuticule de raisin obtenu par la sélection d'au moins un marc de raisin rouge et la concentration des fractions polyphénoliques dudit marc, ledit extrait de cuticule de raisin comprenant au moins 5% en poids, de préférence de 5 à 20% en poids, avantageusement de 5 à 15% en poids, de malvidine-3-O-β-glucoside, et
(ii) le mélange de l'extrait issu de (i) et d'un extrait de propolis.

L'ensemble des différentes caractéristiques ou préférences de la composition selon l'invention présentées pour l'extrait de cuticule de raisin et l'extrait de propolis ainsi que celles relatives à leurs teneurs s'appliquent également au procédé selon l'invention.

La composition selon l'invention peut se présenter sous forme solide ou liquide.

Dans un mode de réalisation particulier, l'invention fournit un kit comprenant :
(a) une première composition comprenant un extrait végétal,
(b) une seconde composition comprenant un extrait de propolis.

L'ensemble des différentes caractéristiques ou préférences de la composition selon l'invention présentées pour l'extrait végétal (a) et l'extrait de propolis (b) ainsi que celles relatives à leurs teneurs s'appliquent également au kit selon l'invention.

Un autre aspect de la présente invention concerne un complément alimentaire comprenant une composition selon l'invention.

Dans un mode de réalisation, le complément alimentaire permet de diminuer, traiter et/ou prévenir des maladies inflammatoires aigues ou chroniques.

Par maladies inflammatoires aigues ou chroniques, on entend toute pathologie ou désordre impliquant des processus inflammatoires aigus ou chroniques.

De nombreuses pathologies humaines impliquant une inflammation sont connues, notamment l'arthrite ; la polyarthrite rhumatoïde ; le lupus érythémateux, en particulier systémique ; la thyroïdite de Hashimoto ; la sclérose multiple, en particulier la sclérose en plaque ; les diabètes, en particulier le diabète auto-immun ; l'uvéite ; dermatites ; psoriasis ; urticaires ; le syndrome néphrotique inflammatoire ; les glomérulonéphrites ; la maladie inflammatoire du colon ; la rectocolite ulcéro-hémorragique ; la maladie coeliaque ; la maladie de Crohn ; le syndrome de Sjogren ; les allergies ; l'asthme ; l'asthme allergique ; les rhinites ; l'eczéma ; l'endométriose ; les maladies liées aux greffes d'organe ou aux réactions du greffon contre l'hôte ; les maladies pulmonaires obstructives chroniques (COPD) ; les bronchites, en particulier les bronchites chroniques ; les maladies de Parkinson ; les maladies d'Alzheimer ou les maladies cutanées inflammatoires.

Dans un mode de réalisation préféré, le complément alimentaire selon l'invention est destiné au traitement et/ou à la prévention des arthrites.

Le complément alimentaire peut en outre comprendre un excipient ou un vecteur inerte, non toxique, par exemple choisi parmi les gélifiants d'origine végétale, de préférence la gélatine.

On pourra citer comme excipient inerte les sucres comme le lactose ou le fructose, la cellulose, le carbonate de calcium, le phosphate tricalcique, le phosphate de magnésium, le stéarate de calcium, le stéarate de magnésium, le talc, la silice colloïdale ou amorphe.

Le complément alimentaire peut également comprendre un composé choisi dans le groupe constitué par les polyols comme la glycérine ou le sorbitol ; les colorants ; les édulcorants comme le sucralose ou les actifs aromatiques comme l'ethylvanilline ou le menthol.

Le complément alimentaire selon l'invention peut se présenter sous forme de poudre, de gélules, de capsules, de gommes à mâcher, de granulés, de granules, de cachets, de pilules, de pastilles, de comprimés ou de tablettes. La mise en oeuvre galénique est effectuée dans des conditions de températures et de pression qui respectent l'intégrité des ingrédients mis en oeuvre et la bioactivité des ingrédients actifs.

Dans le cas où la composition selon l'invention se présente sous la forme d'un complément alimentaire, on administre une dose efficace journalière, dénommée Apport Journalier Recommandé (AJR), de la composition selon l'invention. Par AJR, on entend une dose de la composition selon l'invention consommée sur une période de 24 heures.

Par AJR, on entend notamment une quantité de composition selon l'invention qui comprend entre 100 et 400 mg d'extrait végétal et entre 250 et 1000 mg d'extrait de propolis.

La posologie d'administration du complément alimentaire, par exemple sous forme de gélules, peut varier de 2 à 8 gélules par jour, en fonction de la nature et de la gravité de l'inflammation, à renouveler si besoin.

Un autre aspect de la présente invention concerne une composition à usage topique comprenant une composition selon l'invention pour le traitement et/ou la prévention des maladies inflammatoires aigues ou chroniques, en particulier destinée au traitement et/ou à la prévention des arthrites.

La composition à usage topique peut se présenter sous la forme d'une émulsion eau-dans-huile, huile-dans-eau, eau-dans-huile dans eau, huile-dans-eau dans huile ou d'une formulation anhydre. La composition peut se présenter sous une forme galénique à usage externe comme une crème, une pommade ou un gel.

Selon l'invention, la composition à usage topique est appliquée par étalement sur la ou les régions cutanées présentant une inflammation.

Un autre aspect de la présente invention concerne également un médicament comprenant une composition selon l'invention pour le traitement et/ou la prévention des maladies inflammatoires aigues ou chroniques, en particulier destiné au traitement et/ou à la prévention des arthrites.

L'invention a donc également pour objet l'utilisation de la composition selon l'invention pour précéder, compléter et/ou suivre un traitement thérapeutique ou préventif anti-inflammatoire. Cela signifie que le praticien prescrit au patient qui en a besoin de consommer le complément alimentaire ou le médicament ou d'appliquer la composition à usage topique selon l'invention en cure avant, pendant et/ou après la conduite d'un traitement par anti-inflammatoire.

Un autre aspect de la présente invention concerne un kit comprenant :
- une première composition comprenant un extrait végétal (a) et un extrait de propolis (b),
- une seconde composition comprenant un agent anti-inflammatoire.

L'ensemble des différentes caractéristiques ou préférences de la composition selon l'invention présentées pour l'extrait végétal (a) et l'extrait de propolis (b) ainsi que celles relatives à leurs teneurs et leur ratio s'appliquent également au kit selon l'invention.

Les agents anti-inflammatoires utilisés dans le kit selon l'invention peuvent être choisis parmi les agents anti-inflammatoires stéroïdiens ou non stéroïdiens.

Les différents aspects de l'invention seront mieux compris à la lecture des exemples qui suivent. Ces exemples sont donnés à titre indicatif, sans caractère limitatif.

### Exemple 1 : production d'un extrait de cuticule de raisin

Après avoir sélectionné un marc de raisin rouge parmi des raisins issus de cépages Merlot, Cabernets et Syrah, on procède à une diffusion en présence d'une dispersion aqueuse de dioxyde de soufre.

Une centrifugation avec une gravité comprise entre 7000 et 7500G et un temps de séjour allant de 10 à 20 s est ensuite appliquée au produit ainsi obtenu.

A l'issu de cette centrifugation, on concentre les polyphénols dans le produit par évaporation, le produit ainsi obtenu étant caractérisé par une teneur en matières sèches allant de 30 à 35%.

On procède ensuite à une concentration sous vide en dépression à 50mbar absolu, le concentrat ainsi obtenu étant caractérisé par une teneur en matières sèches supérieure ou égale à 35%.

Le concentrat ainsi obtenu est ensuite déshydraté par atomisation ; on pulvérise ainsi le concentrat en air chaud en légère dépression.

Le produit ainsi obtenu se présente sous forme d'une poudre caractérisée par une teneur en malvidine-3-O-β-glucoside égale à 10% en poids et une humidité relative inférieure à 10%.

### Exemple 2 : production d'un extrait de propolis

Après avoir récolté la propolis brute, celle-ci est mise en solution hydroalcoolique, filtrée, concentrée sous vide puis pasteurisée. Elle est ensuite mélangée à de la poudre de caroube en tant que support puis une étape de séchage par atomisation ("spray-drying") est appliquée au mélange, ledit mélange étant ensuite tamisé. L'extrait de propolis ainsi obtenu se présente sous la forme d'une poudre qui contient 60% de propolis active.

L'extrait de propolis est caractérisé par une teneur moyenne en poids d' acide caféique égale à 0,05%, d'acide férulique égale à 0,001%, de galangine égale à 1,6% et de pinocembrine égale à 1%.

### Exemple 3 : complément alimentaire sous forme de gélule comprenant une composition selon l'invention

| | |
|---|---|
| - Extrait de cuticule de raison⁽¹⁾ | 0,100 g |
| - Extrait de propolis⁽²⁾ | 0,200 g |

| | |
|---|---|
| (1) Anthos-Vin commercialisé par la société Grap'Sud (2) propolis commercialisée par la société Plantex | |

La composition selon l'exemple 3 se présente sous forme de poudre et est conditionnée en gélule et la posologie d'utilisation est de 2 à 4 gélules par jour, à renouveler si besoin.

### Exemples 4 et 5 : compléments alimentaires sous forme de gélule comprenant une composition selon l'invention

| | **Exemple** 4 | **Exemple** 5 |
|---|---|---|
| Extrait de cuticule de raisin⁽¹⁾ | 0,1 g | 0,05 g |
| Extrait de propolis⁽²⁾ | 0,25 g | 0,125 g |
| Gélatine végétale | 0,094 g | 0,094 g |
| Stéarate de magnésium | 0,01 g | 0,01 g |
| Menthol au 1 /11 | 0,01 g | 0,01 g |
| Ethylvanilline | 0,01 g | 0,01 g |
| Silice amorphe précipitée | 3,81 g | 3,81 g |

| | | |
|---|---|---|
| (1) Anthos-vin commercialisé par la société Grap'Sud (2) propolis commercialisée par la société Plantex | | |

La composition selon l'exemple 4 se présente sous forme de poudre et est conditionnée en gélules.

La posologie d'utilisation est de 2 à 6 gélules par jour, à renouveler si besoin.

La composition selon l'exemple 5 se présente sous forme de poudre et est conditionnée en gélule.

La posologie en traitement flash est de 4 gélules toutes les 12 heures pendant les 48 premières heures, puis de 2 gélules toutes les 12 heures pendant les 48 heures suivantes, et si besoin, de 1 à 2 gélules toutes les 12 heures pendant les 48 heures suivantes.

La posologie en traitement préventif est de 1 à 2 gélules, à renouveler si besoin.

### Exemples 6, 7 et 8 : compléments alimentaires sous forme de gélule comprenant une composition selon l'invention

| | **Exemple 6** | **Exemple 7** | **Exemple 8** |
|---|---|---|---|
| Extrait de cuticule de raison⁽¹⁾ | 0,125 g | 0,125 g | 0,125 g |
| Extrait de propolis⁽²⁾ | 0,150 g | 0,150 g | 0,150 g |
| Extrait de poivre noir⁽³⁾ | 0,020 g | | |
| Sulfate de zinc | 0,006 g | | 0,006 g |
| Excipients | QSP | QSP | QSP |

| | | | |
|---|---|---|---|
| (1) Anthos-vin commercialisé par la société Grap'Sud (2) propolis commercialisée par la société Plantex (3) Extrait de poivre noir à 10% de pipérine commercialisé par la société Plantex | | | |

Les compositions selon les exemples 6, 7 et 8 se présentent sous forme de poudre et sont conditionnées en gélule.

Leur posologie d'utilisation en traitement préventif est de 2 gélules par jour pendant 30 jours, à renouveler si besoin.

Leur posologie d'utilisation en traitement flash est de 2 gélules toutes les 12 heures pendant 5 jours.

### Exemple 9 : composition topique sous forme de gel comprenant une composition selon l'invention

| | |
|---|---|
| Solution hydro-alcoolique d'un extrait de cuticule de raisin⁽¹⁾ | 5-10% |
| Solution hydroalcoolique d'un extrait de propolis⁽²⁾ | 2-4% |
| Agents hydratants | 0,1-10% |
| Agents gélifiants | 0,5-20% |
| Eau purifiée + conservateurs | qsp |

| | |
|---|---|
| (1) Anthos-vin commercialisé par la société Grap'Sud (2) propolis commercialisée par la société Plantex | |

La composition est préparée par mélange des solutions hydroalcooliques d'extrait de cuticule de raisin et d'extrait de propolis, puis ajout des agents gélifiants et hydratants et enfin complément par l'eau et conservateurs, pour obtenir la texture souhaitée.

La posologie d'application est de 1 à 4 applications par jour, à renouveler si besoin.

### Exemple 10 : étude de l'hématotoxicité des extraits de cuticule de raisin, de propolis ainsi que d'un extrait de cranberry en tant que composé actif additionnel

Cette étude a pour objectif de montrer que les différents extraits pouvant être incorporés dans une composition selon l'invention n'ont pas d'action toxique sur les cellules humaines.

Des cultures cellulaires et des dosages de cytokines ont été réalisés.

Les trois extraits évalués sont les suivants :
- Extrait de cuticules de raisin (Anthos-vin), resuspendu à 331 mg/ml dans de l'éthanol 100%,
- Extrait de propolis, resuspendue à 500mg/ml dans de l'éthanol 70%,
- Extrait de cranberry, resuspendue à 50mg/ml dans du méthanol 90%.

Les cellules mononuclées humaines ont été isolées à partir de sang périphérique issu de 3 donneurs sains différents. La technique utilisée a été l'isolement sur gradient de Ficoll. Entre Ficoll et plasma s'établit un anneau cellulaire composé des monocytes et des lymphocytes. Celui-ci est récupéré et lavé en PBS (Phosphate Buffered Saline) pour comptage sur Cellule de Malassez.

Les cellules mononuclées sanguines (PBMC, Peripheral Blood Mononuclear Cells) sont cultivées (10⁶/ml) à raison de 10⁵ cellules par puits en plaque 96 puits pour les tests d'hématotoxicité, dans du milieu RPMI (Roswell Park Memorial Institute) supplémenté avec 10% de sérum de veau foetal SVF décomplémenté et des antibiotiques, afin d'éviter une infection bactérienne : pénicilline (100 UI/ml) et streptavidine (100 µg/ml).

Le test de prolifération cellulaire au MTS (3-(4,5-dimethylthiazol-2-yl)-5-(3-carboxymethoxyphenyl)-2-(4-sulfophenyl)-2H-tetrazolium) est une méthode colorimétrique pour déterminer le nombre de cellules viables en prolifération. Le test utilise la transformation du MTS en formazan (produit coloré) qui est soluble dans le milieu de culture cellulaire. L'absorbance du formazan peut être directement lue à 490 nm par un spectrophotomètre. La réduction du MTS en formazan soluble est induite par des déshydrogénases impliquées dans le métabolisme des cellules activées.

Les PBMC sont mises en culture à raison de 10⁵ cellules par puits de plaques 96 puits en condition quiescente (cellules naïves) ou en condition proliférative (en présence de Bacto-Phytohémagglutinine P (PHA, Difco) à 0,5 µg/ml). Le PHA possède une activité mitotique sur les lymphocytes T et induit leur prolifération.

Les extraits ont été testés sur des PBMC provenant de 2 donneurs différents, en quadruplicate, à différentes concentrations : 8, 17, 33 et 66µg/ml pour l'Anthos-vin ; et 5, 20, 50 et 100µg/ml pour la propolis et la cranberry, comparativement à des PBMC sur lesquelles aucun extrait n'a été testé.

Aucun des 3 extraits n'a montré de cytotoxicité sur les cellules hématopoïétiques de deux donneurs en culture, bien qu'elles aient été testées à des concentrations allant jusqu'à 200µg/ml pour les extraits de propolis et de cranberry, et 66µg/ml pour l'extrait de cuticules de raisin.

Aucune toxicité n'a été observée dans les compositions associant extrait de cuticules de raisin et extrait de propolis, ni dans celles associant extrait de cuticules de raisin, extrait de propolis et extrait de cranberry.

### Exemple 11 : étude de l'activité anti-inflammatoire in vitro d'une composition selon l'invention.

Cette étude a pour objectif de démontrer l'activité *in vitro* anti-inflammatoire de compositions selon l'invention, une première composition associant extrait de cuticules de raisin (Anthos-vin) et extrait de propolis et une seconde associant extrait de cuticules de raisin (Anthos-vin), extrait de propolis et extrait de cranberry.

Les cultures cellulaires et les dosages de cytokines ont été réalisés.

Les compositions suivantes ont été testées :

### Compositions selon l'invention :

- une composition comprenant 8 µg/ml d'Anthos-vin et 25 µg/ml d'extrait de propolis,
- une composition comprenant 16 µg/ml d'Anthos-vin et 50 µg/ml d'extrait de propolis,
- une composition comprenant 33 µg/ml d'Anthos-vin et 100 µg/ml d'extrait de propolis,
- une composition comprenant 8 µg/ml d'Anthos-vin, 25 µg/ml d'extrait de propolis et 25 µg/ml d'extrait de cranberry,
- une composition comprenant 16 µg/ml d'Anthos-vin, 50 µg/ml d'extrait de propolis et 50 µg/ml d'extrait de cranberry,
- une composition comprenant 33 µg/ml d'Anthos-vin, 100 µg/ml d'extrait de propolis et 100 µg/ml d'extrait de cranberry,

### Compositions de référence :

- une composition comprenant 8 µg/ml d'Anthos-vin,
- une composition comprenant 16 µg/ml d'Anthos-vin,
- une composition comprenant 33 µg/ml d'Anthos-vin,
- une composition comprenant 25 µg/ml d'extrait de propolis,
- une composition comprenant 50 µg/ml de d'extrait de propolis,
- une composition comprenant 100 g/ml d'extrait de propolis,
- une composition comprenant 25 µg/ml d'extrait de cranberry,
- une composition comprenant 50 µg/ml d'extrait de cranberry,
- une composition comprenant 100 µg/ml d'extrait de cranberry.

Les PBMC ont été isolées à partir de sang périphérique issu de 3 donneurs sains différents. La technique utilisée a été l'isolement sur gradient de Ficoll. Entre Ficoll et plasma s'établit un anneau cellulaire composé des monocytes et des lymphocytes. Celui-ci est récupéré et lavé en PBS pour comptage sur Cellule de Malassez.

Les macrophages des rats ont été isolés par lavage au PBS du péritoine des animaux, centrifugation de celui-ci et lavage en PBS pour comptage sur Cellule de Malassez.

Les PBMC ont été cultivées (10⁶/ml) à raison de 5.10⁵ cellules par puits en plaque 24 puits, dans du milieu RPMI supplémenté avec 10% de SVF décomplémenté et des antibiotiques : pénicilline (100 UI/ml) et streptavidine (100 µg/ml).

Les macrophages de rats ont été cultivés (10⁶/ml) à raison de 5.10⁵ cellules par puits en plaque 24 puits dans du milieu RPMI supplémenté avec 10% de SVF décomplémenté et des antibiotiques pénicilline (100 UI/ml) et streptavidine (100 µg/ml).

Pour induire une réaction inflammatoire sur les PBMC et les macrophages de rats les cellules sont incubées en présence de LPS (LipoPolySaccharide bactérien, produit pro-inflammatoire) à 10µg/ml pendant 48h.

Dosage du NO : après 48h de culture des macrophages péritonéaux de rats, les surnageants ont été prélevés. Le NO possède une haute réactivité chimique. En présence d'eau, ce radical libre est rapidement oxydé de façon stoechiométrique et forme ainsi des ions nitrites (NO₂⁻) selon la réaction :

4NO⁰ + O₂ + H₂O → 4NO₂⁻ + 4H⁺

Les nitrites s'accumulent dans les milieux et sont facilement détectables chimiquement par la méthode de Griess (Kolb *et al.,* 1994) :
- Griess A : Sulfanilamide 1% dans HCl 1,2N,
- Griess B : Naphtylène diamine dichlorhydrate 0,3% dans de l'eau distillée

A 60 µl de Griess A et 60 µl de Griess B, on a ajouté 50 µl de surnageant de culture à doser. La réaction colorimétrique s'est développée à l'abri de la lumière pendant 10 min. Les densités optiques obtenues à 540 nm ont été corrigées par la soustraction des DO obtenues sur les puits contenant le milieu de culture seul.

Dosage des médiateurs de l'inflammation : 48h après l'activation par LPS des PBMC, 25 µl de surnageant cellulaire ont été prélevés et immédiatement testés pour le dosage des cytokines pro- et anti-inflammatoires par la technique du FlowCytomix.

Le FlowCytomix humain est un système d'immunoassay par billes fluorescentes pour la détection quantitative par cytométrie en flux de l'Interféron-γ, l'lnterleukine-1β, l'Interleukine-2, l'Interleukine-4, l'Interleukine-5, l'Interleukine-6, l'Interleukine-8, l'Interleukine-10, l'Interleukine-12p70 et le Tumor Necrosis Factor α et β dans les surnageants de culture.

Des microsphères ont été revêtues avec des anticorps dirigés spécialement envers chaque cytokine détectée par le système multiplex. Les billes peuvent être différenciées par leur taille et par leur émission spectrale. Un mélange de chaque bille revêtue pour chaque cytokine a été incubé avec l'échantillon ou la gamme du standard. Les cytokines présentes dans l'échantillon se lient à l'anticorps fixés à la bille fluorescente. Un second anticorps couplé à la biotine a été ajouté au mélange, cet anticorps spécifique se lie aux cytokines capturées par le premier anticorps.

La streptavidine-phycoérythrine a été ajoutée, elle se lie au conjugué biotine et émet un signal fluorescent.

Dosage de la Prostaglandine E₂: les voies de transduction du signal des eïcosanoïdes sont extrêmement conservées et impliquées dans de nombreux processus physiopathologiques. Les prostaglandines sont synthétisées à partir de l'acide arachidonique par les cyclooxygénases (COX-1 et COX-2), lesquelles convertissent l'acide en prostaglandine H₂ (PGH₂). Ce dernier est alors transformé par les prostaglandines synthases microsomales ou cytosoliques en prostaglandines E₂ (PGE₂) ou en divers autres prostanoïdes.

Le PGE₂ est produit par une large variété de tissus et dans de nombreuses conditions physiopathologiques, dont les inflammations, les arthrites, la fièvre, les tissus lésés, l'endométriose et de nombreux cancers.

Le PGE₂ a été dosé dans les surnageants de culture à l'aide d'un kit de dosage ELISA (Tebu-Bio, Le Perray en Yvelines, France).

Quarante huit heures après l'activation par LPS des PBMC, 100 µl de surnageant cellulaire sont prélevés et immédiatement testés pour le dosage du PGE₂.

Le tableau 1 présente les résultats obtenus et montre les effets des différentes compositions sur le taux des médiateurs pro-inflammatoires.

**Tableau 1**

| **Composés** | **µg/ml** | **TNF-α*** | **IL-1β** | **IL-6** | **PGE2** | **IL-10** | **IL-8** | **IFN-γ** | **NO** |
|---|---|---|---|---|---|---|---|---|---|
| Anthos-vin | 8 | 72 | 70 | 67 | -- | 0 | 51 | 0 | 0 |
| | 16 | 66 | 70 | 67 | -- | 5 | 66 | 0 | 0 |
| | 33 | 63 | 72 | 69 | -- | 41 | 69 | 39 | 0 |
| Extrait de propolis | 25 | 16 | 32 | 27 | -- | 50 | 3 | 100 | 15 |
| | 50 | 55 | 52 | 55 | -- | 100 | 2 | 100 | 55 |
| | 100 | 97 | 87 | 92 | -- | 100 | 27 | 94 | 90 |
| Extrait de cranberry | 25 | 15 | 5 | 7 | -- | 0 | 6 | 5 | 0 |
| | 50 | 20 | 4 | 0 | -- | 0 | 6 | 0 | 0 |
| | 100 | 31 | 9 | 0 | -- | 0 | 11 | 16 | 0 |
| Anthos-vin + extrait de propolis | 8+25 | 0 | 75 | 34 | 40 | 71 | 11 | -- | -- |
| | 16+50 | 50 | 92 | 82 | 64 | 94 | 0 | -- | -- |
| | 33+100 | 73 | 98 | 98 | 94 | 100 | 39 | -- | -- |
| Anthos-vin + extrait de propolis + extrait de cranberry | 8+25+2 5 | 0 | 76 | 72 | 61 | 98 | 0 | 94 | -- |
| | 16+50+ 50 | 100 | 99 | 97 | 76 | 100 | 0 | 100 | -- |
| | 33+100 +100 | 95 | 99 | 99 | 82 | 100 | 41 | 89 | -- |

| | | | | | | | | | |
|---|---|---|---|---|---|---|---|---|---|
| * Pourcentage d'inhibition par rapport aux cellules activées en l'absence des extraits | | | | | | | | | |

Ces résultats montrent que la composition selon l'invention associant Anthos-vin et propolis possèdent une meilleure action anti-inflammatoire globale que celles comprenant l'Anthos-vin ou propolis seuls.

Ces résultats montrent également que la composition selon l'invention associant Anthos-vin, propolis et cranberry possèdent une action significative à l'encontre des TNF-α et IFN-γ, alors que la cranberry seule ne présente que peu d'intérêt pour une action anti-inflammatoire.

Pour confirmer ces résultats, a été testée une composition selon l'invention comprenant (33 µg d'Anthos-vin + 100 µg d'extrait de propolis)/ml sur la transcription des gènes codants pour les molécules inflammatoires chez l'homme.

Les cultures cellulaires et les dosages de cytokines ont été réalisés.

Les cellules mononuclées humaines ont été isolées à partir de sang périphérique issu d'un donneur. La technique utilisée a été l'isolement sur gradient de Ficoll. Entre Ficoll et plasma s'est établi un anneau cellulaire composé des monocytes et des lymphocytes. Celui-ci a été récupéré et lavé en PBS pour comptage sur Cellule de Malassez.

Les PBMC ont été cultivés (10⁶/ml) à raison de 1.10⁷ cellules par puits en plaque 6 puits, dans du milieu RPMI supplémenté avec 10% de SVF décomplémenté et des antibiotiques : pénicilline (100 UI/ml) et streptavidine (100 µg/ml).

Une réaction inflammatoire a été induite sur les PBMC. Rapidement, les cellules ont été incubées en présence de LPS.

La PCR quantitative permet de profiler 84 gènes clés.

Au niveau inflammatoire, la PCR en temps réel permet de mesurer l'expression génique de 84 gènes représentant les principaux médiateurs pro/anti-inflammatoires (chimiokines, récepteurs aux chimiokines, cytokines, récepteurs aux cytokines et gènes reliés au motif inflammatoire), mais aussi ceux impliqués dans la transduction du signal, l'apoptose et le cycle cellulaire. En utilisant la PCR en temps réel, il est possible d'analyser l'expression d'un panel ciblé de gènes en rapport la réaction inflammatoire.

Les gènes ciblés sont listés ci-dessous :
- Molécules des voies de transduction : FOS, HSF1, HSP90AA2, HSP90AB1, HSPB1, JUN, IKBKB, MYC, NFKB1, NOX5, STAT1, STAT3, TANK ;
- Molécules impliquées dans les voies apoptotiques : BAD, BAX, BCL2, CASP10, CASP3, CASP9, TP53 ;
- Molécules d'adhésion : ICAM1, ITGAX, SELE, SELPLG, VCAM1 ;
- Molécules du cycle cellulaire: CCNC, CCND1, CDKN1 B, CDKN2B, MAP2K1, MAPK1, MAPK11 ;
- Chimiokines et Cytokines: CCL3 (MIP-1a), CCL20 (MIP-3a), CXCL9, CXCL10 (IP-10), CXCL11 (I-TAC / IP-9), IFNA2, IFR1, IL10, IL17C, IL1A, IL1B, IL2, IL22, IL4, IL6, IL8, LTA, LTB, MIF, TGFB2, TGFB3 ;
- Récepteurs des Cytokines: IL1RN ;
- Ligands du TNF et Récepteurs: CD40LG, FAS, FASLG, LTA (LT-a), LTB (LT-b), TNF (TNF-a), TNFRSF1 A (TNFR1), TNFRSF10A TNFRSF10B.

Autres facteurs Impliqués dans la Réponse Inflammatoire: C3, CAT, CRP, CYP1A1, CYP2E1, CYP7A1, GPX1, ICEBERG, MMP1, MMP10, MMP3, MMP7, NOS, NOS2A, NOS3, PRKCA, SOD1, SOD2.

L'ARN total des cellules a été extrait à l'aide d'un kit d'extraction (Qiagen, France). Cet ARN a été analysé qualitativement et quantitativement afin de vérifier l'intégralité de ce matériel. Un échantillon d'ARN a été mis à migrer en gel d'agarose 1% à 80V afin de vérifier la présence des ARN ribosomiques 28S et 16S, de l'ARN de transfert et des ARN messagers (mesure qualitative).

De plus, cet ARN a été dosé à 230 nm, 260 nm et 280 nm afin d'être quantifié et de vérifier l'absence de contaminants protéiques.

Un µg d'ARN a été utilisé pour la PCR en temps réel. A l'aide d'un kit (eBioscience, France), l'ARN a été rétro-transcrit en ADNc puis amplifié par PCR. Simultanément à l'amplification, l'utilisation d'une sonde fluorescente (Sybergreen), a permis de mesurer en temps réel l'intégration des bases génomiques. L'ensemble des résultats a été analysé à l'aide d'un logiciel adapté.

Par ailleurs, l'utilisation d'un appareil de PCR en temps réel est nécessaire (Stratagène MX3000P).

Le tableau 2 ci-dessous présente les principales modifications significatives de la transcription des gènes pro-inflammatoire dans les cellules incubées avec la composition selon l'invention par rapport aux cellules non-traitées

**Tableau2**

| **Gènes** | **Noms** | **Up- régulé** | **Down- régulé** |
|---|---|---|---|
| IL1B | **Interleukine 1, Beta (Interleukin 1, beta)** | | -116,81 |
| IL1A | **Interleukine 1, alpha (Interleukin 1, alpha)** | | -57,20 |
| C3 | **Produit du Complément 3 (Complement component 3)** | | -45,82 |
| IL6 | **Interleukine 6 (Interleukin 6 (interferon, beta 2))** | | -40,73 |
| CCL3 | **Ligand de Chimiokine (C-C motif) 3 (Chemokine (C-C motif) ligand 3)** | | -21,53 |
| IL1RN | **Antagoniste du récepteur d'Interleukine 1 (Interleukin 1 receptor antaqonist)** | | -18,74 |
| IL8 | **Interleukine 8 (Interleukin 8)** | | -5,23 |
| TGFB2 | **Facteur transformate ur de croissance, beta 2 (Transformin g growth factor, beta 2)** | +17,78 | |
| HSP90AA2 | **Protéine de choc thermique 90kDa (Heat shock protein 90kDa alpha (cytosolic), class A member 2)** | +12,75 | |
| HSPB1 | **Protéine 1 de choc thermique 27kDa (Heat shock 27kDa protein 1)** | +11,41 | |
| HSP90AB1 | **Protéine 1 de choc thermique 90kDa (Heat shock protein 90kDa alpha (cytosolic), class B member 1)** | +8,77 | |
| TNF | **Facteur nécrosant de tumeur (Tumor necrosis factor (TNF superfamily, member2))** | +8,47 | |
| BAX | **Protéine X associé à BCL-2 (BCL-2 associated X protein)** | +6,24 | |
| IFNA2 | **Interféron , alpha 2 (Interferon, alpha 2)** | +5,99 | |
| SOD1 | **Superoxyde dismutase 1 soluble (Superoxyde dismutase 1, soluble (amyotrophic lateral sclerosis 1 (adult)))** | +5,00 | |
| BCL2 | **Lymphome B-2 (B-cell** | +3,19 | |
| | **CLL/lympho ma 2)** | | |
| IL2 | **Interleukine 2 (Interleukin 2)** | +3,49 | |

Les résultats montrent que les gènes en relation avec l'inflammation sont en majorité inhibés (IL-1, C3, IL-6 et IL-8).

De plus, en présence d'une composition selon l'invention, la transcription du TGFβ2 et de l'interféron-α, qui sont toute deux des molécules bien connues pour leur action anti-inflammatoire, est augmentée.

Par ailleurs, les résultats montrent également que les ARN codant pour les protéines protectrices des cellules (HSP, DAX, BCL-2, SOD1) sont augmentes ce qui confirme l'absence de toxicité de la composition selon l'invention sur les cellules humaines.

### Exemple 12 : étude de l'activité anti-inflammatoire in vivo d'une composition selon l'invention.

Cette étude a été réalisée dans l'objectif de confirmer les résultats de l'étude *in vitro.* Pour cela ont été testées *in vivo* différentes compositions selon l'invention dans le modèle rat arthritique. Ce modèle consiste à développer une crise d'inflammation chronique chez le rat jeune par injection d'antigène bactérien. Ce modèle présente l'avantage de posséder de nombreuses similarités avec les symptômes de la polyarthrite rhumatoïde chez l'homme : une symétrie dans les articulations impliquées, des articulations périphériques qui sont préférentiellement touchées, une hyperplasie synoviale, une infiltration des cellules inflammatoires, des érosions marginales et une meilleure susceptibilité de la maladie chez les femelles. Au contraire de la polyarthrite rhumatoïde humaine, on ne détecte pas de traces des facteurs rhumatoïdes dans le sérum.

Il est à noter que l'état inflammatoire des rats est très avancé (par rapport à l'homme) et nécessite des doses thérapeutiques 10-20 fois supérieures à celles utilisées chez l'homme (tests effectués avec la corticothérapie), en particulier par la voie orale.

Des rats Lewis femelles âgées de 6 semaines ont été immunisés par une injection intradermale, à la base de la queue, de 60 mg de *Mycobacterium butyricum* tués par la chaleur et émulsifiés dans une solution huileuse.

A J+7, une seconde immunisation, dans les mêmes conditions, a été réalisée. Les premiers signes cliniques apparaissent à J+14.

Les rats développent rapidement une arthrite au niveau des articulations périphériques (essentiellement les pattes postérieures) avec une forte réaction inflammatoire de la cavité synoviale se traduisant par une quasi paralysie de l'articulation concernée et une érosion destructive du tissu osseux. Comme dans beaucoup de modèles pathologiques, l'animal subit une forte perte de poids.

Les symptômes inflammatoires sont évalués suivant un score clinique pré-établi et résumé dans le tableau 3 :

**Tableau 3**

| **Score clinique** | **Symptômes** |
|---|---|
| Score 0 | Aucune patte inflammatoire |
| Score 1 | Inflammation/paralysie d'une patte |
| Score 2 | Inflammation/paralysie de deux pattes |
| Score 3 | Inflammation/paralysie de trois pattes |
| Score 4 | Inflammation/paralysie de quatre pattes |

Sept jours avant la 1^{ère} immunisation avec le *Mycobacterium butyricum,* les rats sont séparés en plusieurs groupes :
- Groupe 1 : témoins négatifs, rats sains ;
- Groupe 2 : témoins positifs, rats ayant une arthrite expérimentale sans traitement ;
- Groupe 3 : rats arthritiques recevant 5 doses (une tous les 2 jours) d'une composition selon l'invention comprenant 30 mg d'Anthos-vin et 100 mg d'extrait de propolis par kg d'animal et par dose, dès le 1^{er} jour d'apparition des symptômes ;
- Groupe 4 : rats arthritiques recevant continuellement, tous les 2 jours, une dose d'une composition selon l'invention comprenant 30 mg d'Anthos-vin et 100 mg d'extrait de propolis par kg d'animal et par dose, dès le 1^{er} jour des symptômes et jusqu'à la fin de l'étude (J+50) ;
- Groupe 5 : rats arthritiques recevant 5 doses (une tous les 2 jours) d'une composition selon l'invention comprenant 150 mg d'Anthos-vin et 500 mg d'extrait de propolis par kg d'animal et par dose, dès le 1^{er} jour d'apparition des symptômes ;
- Groupe 6 : rats arthritiques recevant continuellement, tous les 2 jours, une dose d'une composition selon l'invention comprenant 150 mg d'Anthos-vin et 500 mg d'extrait de propolis par kg d'animal et par dose dès le 1^{er} jour des symptômes et jusqu'à la fin de l'étude (J+50).

A partir de l'apparition des symptômes, les rats des groupes 3, 4, 5 et 6 reçoivent par voie orale (gavage) tous les 2 jours leur traitement, soit durant 10 jours (Groupe 3 et 5), soit continuellement (Groupe 4 et 6).

Ils sont pesés quotidiennement et leur score clinique est noté.

Les résultats de cette étude sont présentés dans les tableaux 4 et 5 :

**Tableau 4**

| Groupe de rats | Score cumulatif moyen |
|---|---|
| Groupe 2 | 82 |
| Groupe 3 | 39 |
| Groupe 4 | 6 |
| Groupe 5 | 62 |
| Groupe 6 | 37 |

L'ensemble des rats traités par une des compositions selon l'invention présente des scores cliniques bien inférieurs par rapport aux rats non traités.

Les compositions selon l'invention diminuent fortement la sévérité des signes pathologiques. Le nombre de pattes touchées et l'inflammation de celles-ci sont considérablement réduits.

Il est à noter que les rats recevant des doses modérées de manière continue (groupes 4 et 6) montrent une meilleure réponse par rapport aux autres rats recevant 5 doses (groupes 3 et 5).

Par ailleurs, aucune toxicité apparente n'a été constatée chez les rats des groupes 3, 4, 5 et 6 (absence de saignement, nausée, diarrhée, baisse d'appétit).

**Tableau 5**

| Groupe(s) de rats | Score cumulatif (après 50 jours) |
|---|---|
| Groupe 2 | 327 |
| Groupe 3 + groupe 4 | 111 |
| Groupe 5 + groupe 6 | 208 |

Ces résultats confirment que les compositions selon l'invention testées permettent de diminuer très significativement l'importance des signes pathologiques.

## Revendications

1. Composition anti-inflammatoire comprenant
a. un extrait végétal comprenant au moins 5% en poids, de préférence de 5 à 20% en poids, avantageusement de 5 à 15% en poids, de malvidine-3-O-β-glucoside ; et
b. un extrait de propolis comprenant de l'acide caféique ou un de ses esters, de l'acide férulique, de la galangine et de la pinocembrine ;
en un ratio en poids a/b extrait végétal/extrait de propolis allant de 1/4 à 4/1.

2. Composition selon la revendication 1 qui est sous forme solide ou sous forme liquide ou pour laquelle l'extrait végétal est un extrait de cuticule de raisin, obtenu ou susceptible d'être obtenu par un procédé comprenant la concentration des fractions polyphénoliques d'au moins un marc de raisin rouge.

3. Composition selon les revendications précédentes comprenant
- de 0,1 à 50% en poids, de préférence de 1 à 40% en poids, d'extrait végétal, ou
- de 20 à 80% en poids, de préférence de 30 à 70% en poids, d'extrait de propolis, ou
- de 0,1 à 50% en poids, de préférence de 1 à 40% en poids, d'extrait végétal et de 20 à 80% en poids, de préférence de 30 à 70% en poids, d'extrait de propolis.

4. Composition selon les revendications précédentes pour laquelle l'extrait de propolis comprend
• de 0,001 à 0,2% en poids, de préférence de 0,005 à 0,15% en poids, d'acide caféique ou d'un de ses esters ;
• de 0,0001 à 0,005% en poids, de préférence de 0,0005 à 0,003% en poids, d'acide férulique ;
• de entre 0,05 à 10% en poids, de préférence de 0,1 à 6% en poids, de galangine ;
• de 0,1 à 5% en poids, de préférence de 0,2 à 3% en poids, de pinocembrine.

5. Composition selon les revendications précédentes comprenant également de la capsaïcine ou un agent anti-inflammatoire ou leur mélange.

6. Composition selon la revendication précédente comprenant de 0,01 à 0,1%, en poids, de préférence de 0,025 à 0,075% en poids, de capsaïcine.

7. Composition selon l'une quelconque des revendications précédentes comprenant également un ingrédient alimentaire.

8. Composition selon la revendication précédente pour laquelle l'ingrédient alimentaire est choisi parmi la pipérine ou un dérivé du zinc.

9. Composition selon la revendication 7 ou 8 comprenant de 0,01 à 4% en poids, de préférence de 0,1 à 1% en poids, de pipérine.

10. Composition selon la revendication 7 ou 8 comprenant de 0,01 à 2% en poids, de préférence de 0,1 à 1,5 % en poids, d'un dérivé de zinc.

11. Complément alimentaire comprenant une composition selon l'une quelconque des revendications 1 à 10.

12. Composition selon l'une quelconque des revendications 1 à 6, sous la forme d'une composition topique.

13. Composition selon la revendication 12, pour utilisation pour le traitement ou la prévention des maladies inflammatoires aiguës ou chroniques, en particulier pour le traitement ou la prévention des arthrites.

14. Composition selon l'une quelconque des revendications 1 à 6, destinée à être utilisée comme médicament.

15. Composition selon l'une quelconque des revendications 1 à 6, pour utilisation pour le traitement ou la prévention des maladies inflammatoires aiguës ou chroniques, en particulier pour le traitement ou la prévention des arthrites.

16. Kit comprenant :
- une première composition selon l'une quelconque des revendications 1 à 6,
- une seconde composition comprenant un agent anti-inflammatoire.

17. Procédé de préparation d'une composition selon les revendications 2 à 10 comprenant :
(i) la préparation d'un extrait de cuticule de raisin obtenu par la sélection d'au moins un marc de raisin rouge et la concentration des fractions polyphénoliques dudit marc,
(ii) le mélange de l'extrait issu de (i) et d'un extrait de propolis.

## Patentansprüche

1. Entzündungshemmende Zusammensetzung, enthaltend
a. einen Pflanzenextrakt, der mindestens 5 Gew.-%, vorzugsweise 5 bis 20 Gew.-%, vorteilhafterweise 5 bis 15 Gew.-% an Malvidin-3-O-β-Glucosid enthält; und
b. einen Propolisextrakt, der Kaffeesäure oder eines ihrer Ester, Ferulasäure, Galagin und Pinocembrin enthält;
in einem Gewichtsverhältnis a/b Pflanzenextrakt/Propolis-Extrakt, das von 1/4 bis 4/1 geht.

2. Zusammensetzung nach Anspruch 1, die in fester Form oder flüssiger Form vorhanden ist oder für die der Pflanzenextrakt ein Traubenhautextrakt ist, der erhalten wird oder geeignet ist, durch einen Prozess erhalten zu werden, der die Konzentration von Polyphenolfraktionen mindestens eines Tresters von roten Trauben umfasst.

3. Zusammensetzung nach den vorhergehenden Ansprüchen, enthaltend
- 0,1 bis 50 Gew.-%, vorzugsweise von 1 bis 40 Gew.-% Pflanzenextrakt, oder
- 20 bis 80 Gew.-%, vorzugsweise 30 bis 70 Gew.-% Propolis-Extrakt, oder
- 0,1 bis 50 Gew.-%, vorzugsweise 1 bis 40 Gew.-% Pflanzenextrakt und 20 bis 80 Gew.-%, vorzugsweise 30 bis 70 Gew.-% Propolis-Extrakt.

4. Zusammensetzung nach den vorhergehenden Ansprüchen, für die der Propolis-Extrakt
• von 0,001 bis 0,2 Gew.-%, vorzugsweise von 0,005 bis 0,15 Gew.-% Kaffeesäure oder eines ihrer Ester;
• von 0,0001 bis 0,005 Gew.-%, vorzugsweise von 0,0005 bis 0,003 Gew.-% Ferulasäure;
• von zwischen 0,05 bis 10 Gew.-%, vorzugsweise von 0,1 bis 6 Gew.-% Galangin;
• von 0,1 bis 5 Gew.-%, vorzugsweise von 0,2 bis 3 Gew.-% Pinocembrin
enthält.

5. Zusammensetzung nach den vorhergehenden Ansprüchen, gleichfalls enthaltend Capsaicin oder ein entzündungshemmendes Agens oder ihre Mischung.

6. Zusammensetzung nach dem vorhergehenden Anspruch, enthaltend von 0,01 bis 0,1 Gew.-%, vorzugsweise von 0,025 bis 0,075 Gew.-% an Capsaicin.

7. Zusammensetzung nach einem beliebigen der vorhergehenden Ansprüche, gleichfalls enthaltend einen Lebensmittelbestandteil.

8. Zusammensetzung nach dem vorhergehenden Anspruch, für die der Lebensmittelbestand ausgewählt ist aus Piperin oder Zinkderivat.

9. Zusammensetzung nach Anspruch 7 oder 8, enthaltend von 0,01 bis 4 Gew.-%, vorzugsweise von 0,1 bis 1 Gew.-% an Piperin.

10. Zusammensetzung nach Anspruch 7 oder 8, enthaltend von 0,01 bis 2 Gew.-%, vorzugsweise von 0,1 bis 1,5 Gew.-% eines Zinkderivats.

11. Nahrungsergänzungsmittel, enthaltend eine Zusammensetzung nach einem der Ansprüche 1 bis 10.

12. Zusammensetzung nach einem beliebigen der Ansprüche 1 bis 6 in der Form einer topischen Zusammensetzung.

13. Zusammensetzung nach Anspruch 12 für die Verwendung für eine Behandlung oder Prävention von akuten oder chronischen Entzündungskrankheiten, insbesondere für die Behandlung oder die Prävention von Gelenkentzündungen.

14. Zusammensetzung nach einem beliebigen der Ansprüche 1 bis 6, die vorgesehen ist als Medikament verwendet zu werden.

15. Zusammensetzung nach einem beliebigen der Ansprüche 1 bis 6 für die Verwendung der Behandlung oder der Prävention von akuten und chronischen Entzündungskrankheiten, insbesondere für die Behandlung oder die Prävention von Gelenkentzündungen.

16. Kit, enthaltend:
- eine erste Zusammensetzung nach einem beliebigen der Ansprüche 1 bis 6,
- eine zweite Zusammensetzung enthaltend ein entzündungshemmendes Agens.

17. Verfahren zur Herstellung einer Zusammensetzung nach den Ansprüchen 2 bis 10, enthaltend:
(i) die Herstellung eines Traubenhautextrakts, der durch die Wahl von mindestens einem Trester von roten Trauben und der Konzentration von Polyphenolfraktionen des Tresters erhalten wird,
(ii) die Mischung des sich von (i) ergebenden Extrakts und eines Propolis-Extrakts.

## Claims

1. An anti-inflammatory composition comprising
a. a plant extract comprising at least 5% by weight, preferably from 5 to 20% by weight, advantageously from 5 to 15% by weight of malvidine-3-O-β-glucoside; and
b. a propolis extract comprising caffeic acid or one of its esters, ferulic acid, garangin and pinocembrin;
and a plant extract/propolis extract a/b weight ratio ranging from 1/4 to 4/1.

2. The composition according to claim 1, which is under solid form or liquid form or for which the plant extract is a grape cuticle extract, obtained or able to be obtained by a method comprising the concentration of polyphenolic fractions of at least a red grape marc.

3. The composition according to the preceding claims, comprising:
- from 0.1 to 50% by weight, preferably from 1 to 40% by weight, of a plant extract, or
- from 20 to 80% by weight, preferably from 30 to 70 % by weight, of propolis extract, or
- from 0.1 to 50% by weight, preferably from 1 to 40% by weight, of plant extract and from 20 to 80% by weight, preferably from 30 to 70% by weight of propolis extract.

4. The composition according to the preceding claims for which the propolis extract comprises
• from 0.001 to 0.2% by weight, preferably from 0.005 to 0.15% by weight, of caffeic acid or of one of its esters;
• from 0.0001 to 0.005% by weight, preferably from 0.0005 to 0.003% by weight of ferulic acid;
• from between 0.05 to 10% by weight, preferably from 0.1 to 6% by weight, of galangin;
• from 0.1 to 5% by weight, preferably from 0.2 to 3% by weight, of pinocembrin.

5. The composition according to the preceding claims also comprising capsaicin or an anti-inflammatory agent or a mixture thereof.

6. The composition according to the preceding claim comprising from 0.01 to 0.1%, by weight, preferably from 0.025 to 0.075% by weight of capsaicin.

7. The composition according to any of the preceding claims also comprising a food ingredient.

8. The composition according to the preceding claim, for which the food ingredient is selected from piperine or a zinc derivative.

9. The composition according to claims 7 or 8 comprising from 0.01 to 4% by weight, preferably from 0.1 to 1% by weight of piperine.

10. The composition according to claims 7 or 8 comprising from 0.01 to 2% by weight, preferably from 0.1 to 1.5 % by weight of a zinc derivative.

11. A food supplement comprising a composition according to any one of claims 1 to 10.

12. The composition according to any one of claims 1 to 6 under the form of a topical composition.

13. The composition according to claim 12 for use for treating or preventing acute or chronic inflammatory diseases, in particular for treating or preventing arthritises.

14. The composition according to any one of claims 1 to 6, intended to be used as a medicament.

15. The composition according to any one of claims 1 to 6 for use for treating or preventing acute or chronic inflammatory diseases, in particular for treating or preventing arthritises.

16. A kit comprising:
- a first composition according to any of claims 1 to 6,
- a second composition comprising an anti-inflammatory agent.

17. A method for preparing a composition according to claims 2 to 10, comprising:
(i) preparing a grape cuticle extract obtained by selecting at least one red grape marc and concentrating the polyphenolic fractions of said marc,
(ii) mixing the extract from (i) and a propolis extract.
